# EUROPEAN PATENT SPECIFICATION

(11) **EP 2 298 248 B1**
(45) Date of publication and mention of the grant of the patent: **19.04.2017**
(21) Application number: 09749404.1
(22) Date of filing: 20.05.2009
(51) Int. Cl.: A61F 2/962

(54) **STENT GRAFT WITH OPENING AND METHOD FOR BINDING THE STENT GRAFT**
STENTGRAFTPROTHESE MIT ÖFFNUNG UND VERFAHREN ZUR BINDUNG DER STENTGRAFTPROTHESE
GREFFE DE STENT AVEC OUVERTURE ET PROCÉDÉ DE FIXATION DE LA GREFFE DE STENT

(30) Priority: 21.05.2008 CN 200810037753
(43) Date of publication of application: 23.03.2011
(73) Proprietor: MicroPort Endovascular (Shanghai) Co., Ltd., Pudong New District Shanghai Shanghai 201318 (CN)
(72) Inventor: ZHU, Qing, Pudong New District Shanghai 201203 (CN); LUO, Qiyi, Pudong New District Shanghai 201203 (CN); YUAN, Zhenyu, Pudong New District Shanghai 201203 (CN); GAO, Yanbin, Pudong New District Shanghai 201203 (CN)
(74) Representative: Dennemeyer & Associates S.A.
(86) International application number: PCT/CN2009/000552
(87) International publication number: WO 2009/140861

(56) References cited:
- WO-A1-03/022181
- WO-A1-2005/034808
- WO-A1-2005/122962
- CN-A- 1 867 301
- CN-A- 1 867 302
- CN-A- 101 283 937
- CN-Y- 2 822 554

## Description

### FIELD OF THE INVENTION

The present invention relates to a method for binding a stent graft, comprising a binding thread and a control wire.

### BACKGROUND OF THE INVENTION

Abdominal aortic aneurysm (AAA) means diseases or lesions of the arterial wail result in abdominal aortic being local or diffuse swelling, with the main symptom of pulsatile mass. Infra-renal AAA is common, which may involve one side or both sides of the coax artery. AAA can be classified pathologically as true aneurysm, pseudoaneurysm and dissecting aneurysm. Once the aneurysm rupture occurs, the mural thrombus in the aneurysm will drop and embolize the artery, so the patient needs first aid. Abdominal aortic aneurysm is one of the common diseases which imperil wrinkly and elderly people's life. The prevalence of abdominal aortic aneurysm increases year after year along with the population aging, and the increase of the prevalence of diseases such as diabetes, atherosclerosis and hypertension etc.

With respect to the traditional open-mode surgery, the risk factors affecting the surgery mainly lie in the elderly, serious heart and lung diseases, as well as renal inadequacy. Generally, the patients of abdominal aortic aneurysm are elderly peoples who suffer from coronary heart disease, hypertension, diabetes, cerebral thrombosis, chronic obstructive lung disease and renal inadequacy. Surgical treatment may be safe only after active treatment of the complication before the surgery, and under better cardiopulmonary function and stable blood pressure and blood glucose.

The treatment of abdominal aortic aneurysm using stent graft endovascular exclusion can obtain good effect. Compared to common open-mode surgery, the advantages of the endovascular exclusion are less trauma, faster recovery after treatment, less complications and significantly shorter hospitalization time. Specifically, it provides the treatment opportunity for the high-risk patients who suffer from serious complications and can not tolerate the traditional surgery. However, there are certain contraindications for the treatment of abdominal aortic aneurysm with the traditional stent, such as short aneurysmal neck, the length of less than 15 mm, proximal end aneurysmal neck with an angle greater than 60°, retortion of the taper aneurism, and involvement of the visceral artery. Short aneurysmal neck may result in insufficient anchorage zone at the proximal end of the stent, which causes the displacement of the stent; and the involvement of the visceral artery can not be isolated by the stent graft. According to the above contraindications, about 30% to 40% of the patients can not be treated with common stent graft. Under this condition, a stent graft with an opening presents its advantages.

The present stent with side opening has been formed one or more openings at the proximal end. After the release of the stent graft, the openings are aligned to the openings of important branch vessels such as renal artery, mesenteric artery and coeliac trunk artery, such that it is possible to use the area between the renal artery and the coeliac trunk artery or other important branch vessel as the aneurysmal neck to locate the stent. Thus the above-mentioned problem is resolved.

However, such stent graft also has the following problem: the stent graft can not rotate freely for location before it being released, which results in certain difficulty for the accurate alignment between the openings of the stent and the openings of the renal artery, thus effects the success rate of the surgery.

A stent graft with an opening which can rotate freely for location before the stent being released is advantageous for the accurate alignment between the opening of the stent and the opening of the renal artery and increases the success rate of the surgery.

Such a stent graft with an opening comprises multiple annular wave-shaped stent sections and a graft pipe sutured with these stent sections, wherein the graft pipe has at least one opening where an opening retaining wire is sutured for supporting the opening.

In said stent graft, the graft pipe is formed with a U-shape opening at the head or proximal end, the wave-shaped stent section at this end itself is used as the opening retaining wire for supporting the opening. Moreover, the graft pipe is formed with a round or oval opening at the side thereof and the opening retaining wire is made of NiTi alloy wire. Said opening retaining wire may be sutured in the shape of circle, oval, bracket, diamond or flying saucer.

A stent graft with an opening which is similar to the one outlined above has been described in the international patent application WO 2005/034808 A1.

A method for binding a stent graft has been described in the international patent application WO 03/022181 A1. There, the self-expanding stent is retained in a reduced profile configuration by internally mounted members which engage a catheter shaft. These members extend between two ends. Said ends are each engaged to a strut or other portion of the stent in such a manner that when the member is looped over the catheter shaft, the members retain the stent in the reduced profile configuration and prevent the stent from self-expanding.

### BRIEF DESCRIPTION OF THE INVENTION

The object of the present invention is to provide an alternative method for binding the stent graft. Using such binding method, it is possible to make the stent rotate freely for location before it is released. Such method is advantageous for the accurate alignment between the opening of the stent and the opening of the renal artery and increases the success rate of the surgery.

The present invention discloses a method for binding the stent graft, comprising a binding thread and a control wire and comprising the steps of: suturing coils on the stent sections of the stent graft; binding a double-strand binding thread onto the stent graft by winding it circumferentially and through the coils; and placing the control wire through two ends of the double-strand binding thread.

In said method for binding the stent graft, the double-strand binding thread is wound around the stent graft circumferentially for one circle and hold in position by means of a control wire passing through the overlapping circle formed by the head end and tail end of the thread, allowing the diameter of the stent graft is limited to a circle whose perimeter is the length of the binding thread.

In said method for binding the stent graft, the step of suturing coils on the stent sections of the stent graft further comprises: said coils may be sutured on the edge of the stent sections.

In said method for binding the stent graft, the step of suturing coils on the stent sections of the stent graft further comprises: said coils may be sutured on the middle position of the edge of the stent sections.

In said method for binding the stent graft, for the stent with small diameter, the double-strand binding thread is used to bind the apex of the edge on the first section of the graft.

In said method for binding the stent graft, on the stent section at the distal end, one coil is sutured on the apex of an edge, or 2-3 coils are sutured on the apexes of adjacent edges.

In said method for binding the stent graft, the step of suturing coils on the stent sections of the stent graft further comprises: said coils are sutured on each edge or every other edge of the stent sections.

In said method for binding the stent graft, the step of suturing a coil on the stent sections of the stent graft further comprises: each of said double-strand binding thread may have the same length or different length.

In said method for binding the stent graft, said double-strand binding thread may be one closed thread, or may be a single strand with a coil at both ends or double strands

In said method for binding the stent graft, the ejection pipe in the delivery system used to deliver and eject the stent graft is also sutured with a coil.

Said method for binding the stent graft further comprises a step of release of the stent graft: under initial state, the stent graft is gripped with pressure within the outer pipe so that its diameter is reduced to minimum, and during release, the outer pipe is drawn out first, then the stent graft is released from the out pipe, the diameter increases to the size controlled by the binding thread; when the stent is released from the out pipe, it is in the pre-released state due to the restriction of the binding thread, and the last section of the stent is connected with the head end of the ejection pipe by the binding thread and the control wire so that the stent graft may rotate with the rotation of the ejection pipe; the control wire is drawn out and the binding thread is loosened after the alignment of the openings, as a result, and the stent graft expands spontaneously to the size of the blood vessel due to self-elasticity.

The present invention has the following advantageous effects compared to the prior art: The binding method of the present invention binds the double strands binding thread onto the middle position of the stent sections of the stent graft through the coil along the circumferential direction, and then binds the two ends of the binding thread with the control wire. Comparing to prior art, this new binding method makes the stent can rotate freely for location before it being released, which is advantageous for the accurate alignment between the opening of the stent and the opening of the artery.

### BRIEF DESCRIPTION OF THE DRAWINGS

- **Figure 1**: is a schematic view of the structure of a stent graft with an opening;
- **Figure 2**: is an enlarged view of the part of the side opening;
- **Figure 3A**: illustrates various shapes of the retaining loop for the opening;
- **Figure 3B**: is a schematic view of the stent section with a U-shaped opening;
- **Figure 4**: illustrates the method for binding the stent graft of the present invention;
- **Figures 5A-5C**: is a schematic view of the structure of the binding threads;
- **Figure 6**: illustrates the connection method of the ejection pipe and the distal end of the stent;
- **Figures 7A-7C**: illustrate the release methods of the stent graft.

### DETAILED DESCRIPTION OF THE INVENTION

Further description of the present invention will be given as below accompanying with the drawings and the embodiments.

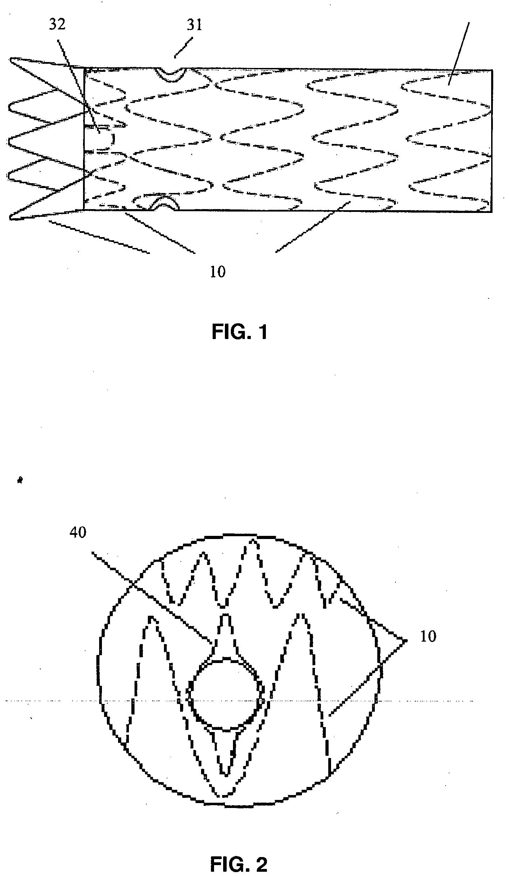

Figure 1 illustrates the structure of a stent graft with an opening. Referring to Figure 1, stent graft 1 comprises a graft pipe 20 made of macromolecule material and wave-shaped NiTi wire stent sections 10 sutured on the graft pipe 20. The NiTi wires 10 shown by real lines in Figure 1 are sutured to and outside the graft pipe 20, and the NiTi wires 10 shown by broken line in Figure 1 are sutured to and inside the graft pipe 20. According to the specific site of the patients' renal artery, one or more openings are formed on the graft pipe 20 as an access of the renal artery or other main blood vessels. These openings include a side opening 31 and a U-shaped opening 32.

Figure 2 is an enlarged view of the side opening 31. The side opening 31 is sutured with an opening retaining wire 40 made of NiTi wire. The opening retaining wire 40 may support the opening, and prevent the circumferential or oval side opening 31 from deforming or collapsing under an external force, because the forming or collapsing may block the access of the blood flow. The other parts of Figure 2 are wave-shaped stent sections 10. The opening retaining wire 40 may also be made of other metallic material.

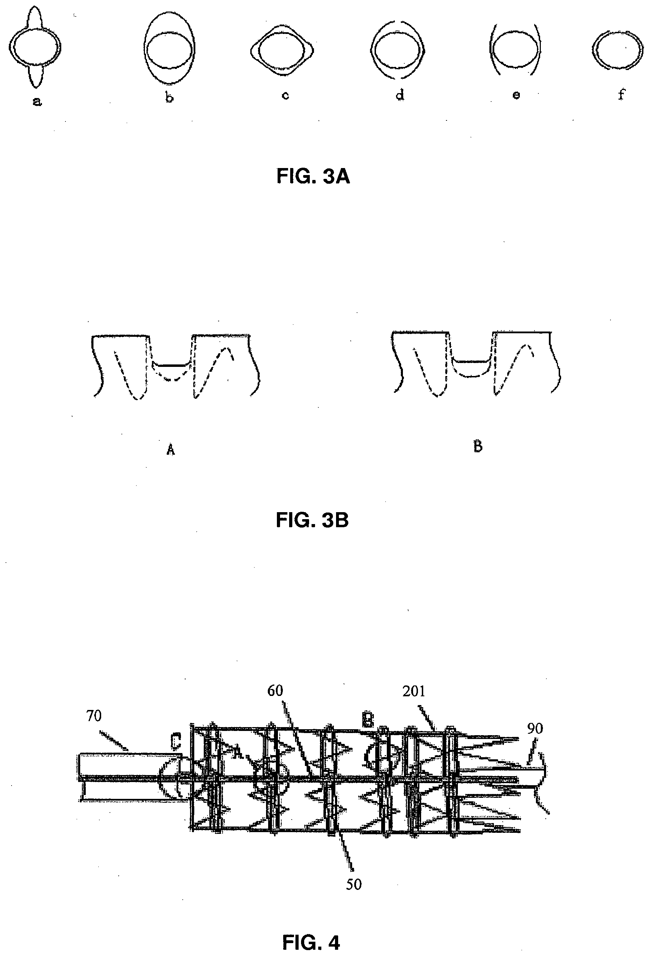

Graft pipe 20 is formed with openings 32 at the head or proximal end where wave-shaped stent sections 10 having special shapes themselves are used as the opening retaining wires for supporting. Such special shapes of the wave-shaped stent sections 10 are shown in Figures 3A-B, in which the middle real line in Figure 3B represents the U-shaped opening cut from the edge of the graft pipe 20, and the broken lines in Figure 3B represent the Ni-Ti wire. Referring to Figure 3A, the opening retaining wire 40 may be sutured in various forms such as circle, oval, bracket, diamond, flying saucer, etc. The circles in Figure 3A represent the opening on the graft pipe 20, the patterns outside the opening represent the opening retaining wires with different shapes.

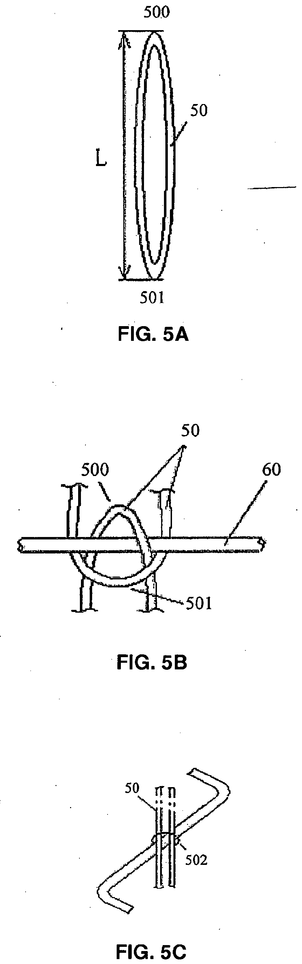

Figure 4 illustrates the method for binding the stent graft of the present invention. Referring to Figure 4 and in conjunction with Figures 5A-5C, first, a coil 502 is sutured onto the stent sections 10 of the stent graft 1; secondly, a double-strand binding thread 50 is bound onto the stent graft 1 by winding it along the circumferential direction and passing through the coil; last, a control wire is passed though two ends of the double-strand binding thread 50. Referring back to Figures 5A-5C, wherein the double-strand binding thread 50 is knotted by a macromolecule thread to form double strands, and has a length less than the circumference of the graft pipe under extended state. Then the double-strand binding thread 50 is wound around the stent graft 1 circumferentially for one circle and hold in position by means of a control wire 60 passing through the overlapping circle formed by the head end 500 and tail end 501 of the thread 50 together, as a result, the stent graft 1 is compacted with its diameter being limited to a circle whose perimeter is substantially equal to the length L of the binding thread 50. Each double-strand binding thread may have the same length or different length. The double-strand binding thread may be one closed thread, or may be a single strand with a coil at both ends or double strands.

The methods for suturing the coil 502 on the stent sections 10 of the stent graft 1 comprise: suturing said coil on an edge of the stent sections 10, and preferably on the middle position of the edge of the stent sections 10; suturing said coil on each edge or every other edge of the stent sections 10. On the stent section at the distal end, there is one coil 502 sutured on the apex of the edge, or 2-3 coils 502 sutured on the apexes of the adjacent edges.

The method for binding the double-strand binding thread on the middle position of the edge of the stent sections by the coil further comprises: first, forming an circle 502 having a diameter of 1-3 mm with a suture thread on the middle position of each edge of the stent sections, then passing the binding thread 50 through the circle 502 to fix the binding thread 50 on the middle position of the stent sections.

Again referring to Figure 4, both ends of the binding thread 50 are further fixed with the control wire 60. After bound in the above manners, all the stent sections are compressed until the diameters thereof are minimum so as to be encased in the outer pipe. Thus the binding process is over.

In the binding process, the stent sections of the stent graft at different positions may adopt different binding ratios according to the inner diameter of the blood vessel. The diameter of the stent can be restricted to 30%-100% of its original size by adjusting the length L of the binding thread, which ensuring the stent can rotate and move freely to adjust the position under the partly pre-released state in the blood vessel cavity, as well as ensuring a guide wire and a catheter can easily pass though the stent under restricted-state.

In the binding process, for the big stent (with the diameter of greater than 20 cm), macromolecule sleeve is used to restrict the exposed section at the proximal end. When partly released, the macromolecule sleeve can restrict the exposed section from being loosened so as to allow the stent to rotate circumferentially, or the exposed section can not move if it is expanded. For the small stent (with the diameter of less than 20 cm), it is generally equipped at its proximal end with an exposed stent section (no graft). By the method of using the double-strand binding thread to bind the edge apex of the first section of the graft 201 (see Figure 4) (the edge apex of the first section of the graft means the edge apex of the first section of the proximal end of the stent graft), the problem that a sleeve can not be employed for restricting the small-diameter stent whose exposed section is too short and tends to spring out of the sleeve is solved.

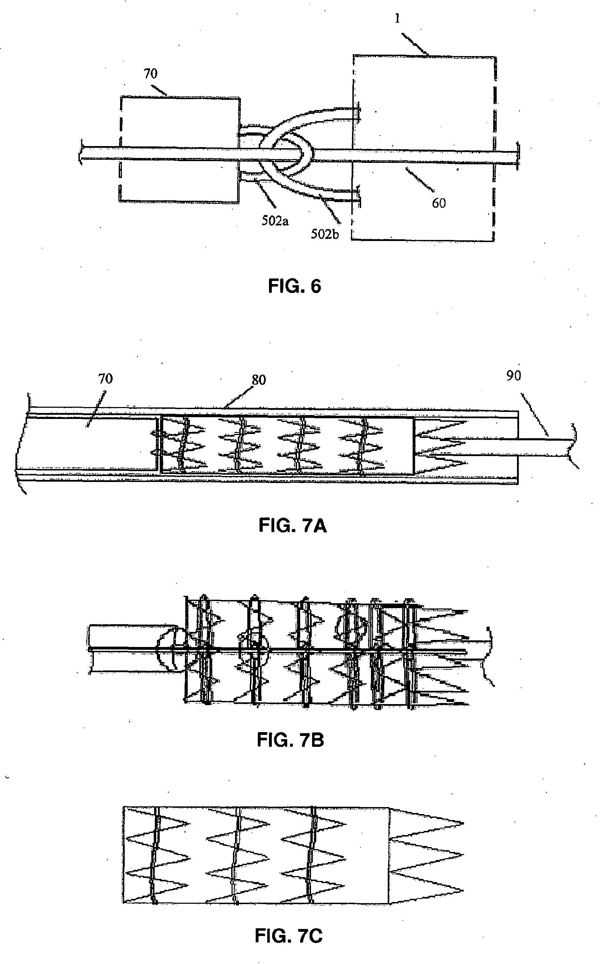

The ejection pipe 70 in the delivery system for delivering and pushing out the stent graft is also sutured with a coil. During the binding process, under the condition that the distal end of the stent graft 1 is connected with the head part of the ejection pipe 70, as shown in Figure 6, the binding coil 502a on the ejection pipe 70 is overlapped into the binding coil 502b on the stent graft 1, then the control wire 60 is passed though from the binding coil 502a, as a result, the stent graft 1 is connected with the ejection pipe 70. When the stent 1 is partly pre-released, it is possible to rotate the ejection pipe 70 circumferentially in a range of 360° to bring the stent 1 to move for adjusting the position.

Figure 7 illustrates the corresponding release method of the binding method. Referring to Figures 7A-7C, under initial state, the stent graft 1 is fully gripped within the outer pipe 80 and restricted to have a minimum diameter, i.e., the inner diameter of the outer pipe, as shown in Figure 7A. When the stent graft is released, first, the outer pipe 80 is drawn out, then the graft is released from the out pipe 80 until the diameter thereof increases to the size controlled by the binding thread, as shown in Figure 7B. In this embodiment, the stent is in the pre-released state due to the restriction of the binding thread, and the stent sections are bound along the circumferential direction using the new binding method in which the binding threads with different length can be used if necessary, so that the diameter of the stent is evenly reduced, the problem of displacement of the relative position of the two side openings when bound with other method is avoided, and it is easier for the alignment of the stent with more than two openings of the blood vessel. After the alignment, the control wire is drawn out, then the diameter of the graft is recovered to the size of the blood vessel implanted, as shown as Figure 7C.

When the stent is released from the outer pipe 80, it is in the pre-released state and has a diameter less than that in the natural state due to the restriction of the binding thread. The last section of the stent is connected with the head end of the ejection pipe 70 by the binding thread and the control wire, so that the stent graft may rotate with the rotation of the ejection pipe. After the side openings of the stent graft are aligned with the openings of the blood vessel, the control wire is drawn out and the binding thread is loosened, as a result, the stent graft expands spontaneously to the size of the blood vessel due to self-elasticity. The stent is left in the patient's body after the carrier is removed.

The above embodiments are provided to one skilled in the art to carry out or use the present invention, and one skilled in the art will recognize that various modifications and changes may be made to the embodiments described without departure from the scope of the invention. So the protection scope of the present invention is not limited to the above-mentioned embodiments, whereas it should be the largest scope of the inventive feature disclosed in the Claims.

## Claims

1. A method for binding a stent graft (1) comprising a binding thread (50) and a control wire (60),
**characterized in that** comprising: suturing coils (502) on the stent sections (10) of the stent graft (1);
binding circumferentially a double strand binding thread (50) through the coils (502) onto the stent graft (1); and
setting the control wire (60) through both ends of the double-strand binding thread (50).

2. The method for binding a stent graft according to claims 1, **characterized in that** after winding around the stent graft one circle, the double-strand binding thread is held in position by the control wire extending through an overlapping circle that is formed by the head end and the tail end of the binding thread, so that the diameter of the stent graft is limited to a circle whose perimeter is the length of the binding thread.

3. The method for binding a stent graft according to claim 1, **characterized in that** the step of suturing coils on the stent sections of the stent graft further comprises:
said coils may be sutured on the edges of the stent sections.

4. The method for binding a stent graft according to claim 3, **characterized in that** the step of suturing a coil on the stent sections of the stent graft further comprises:
said coil may be sutured on the middle position of the edges of the stent sections.

5. The method for binding a stent graft according to claim 3, **characterized in that** for the stent graft with small diameter, the double-strand binding thread is used to bind the apex of the edge on the first section of the graft pipe.

6. The method for binding a stent graft according to claim 3, **characterized in that** the apex of the edge on the stent section at its distal end is sutured with one coil, or the apexes of the adjacent edges are sutured with 2-3 coils.

7. The method for binding a stent graft according to claim 3, **characterized in that** the step of suturing coils on the stent sections of the stent graft further comprises: said coils are sutured on each edge or every other edge of the stent sections.

8. The method for binding a stent graft according to any one of claims 1 to 7, **characterized in that** each of the double-strand binding threads may have the same length or different length.

9. The method for binding a stent graft according to any one of claims 1 to 7, **characterized in that** the double-strands binding thread may be one closed thread, or may be a single strand with a coil at both ends or double strands.

10. The method for binding a stent graft according to claim 6, **characterized in that** the ejection pipe in the delivery system used to deliver and eject the stent graft is also sutured with a coil.

## Patentansprüche

1. Verfahren zum Binden einer Stentstütze (1), umfassend einen Bindedraht (50) und einen Steuerdraht (60),
**gekennzeichnet durch** folgende Schritte:
Vernähen von Wicklungen (502) auf den Stentabschnitten (10) der Stentstütze (1);
umfangsmäßiges Binden eines doppelsträngigen Bindedrahts (50) durch die Wicklungen (502) hindurch auf die Stentstütze (1); und
Legen des Steuerdrahts (60) **durch** die beiden Enden des doppelsträngigen Bindedrahts (50) hindurch.

2. Verfahren zum Binden einer Stentstütze nach Anspruch 1, **dadurch gekennzeichnet, dass** nach dem Wickeln eines Kreises um die Stentstütze herum der doppelsträngige Bindedraht durch den Steuerdraht an Ort und Stelle gehalten wird, der sich durch einen überlappenden Kreis erstreckt, der durch das Anfangsende und das Abschlussende des Bindedrahts gebildet wird, sodass der Durchmesser der Stentstütze auf einen Kreis eingeschränkt ist, dessen Umfang die Länge des Bindedrahts ist.

3. Verfahren zum Binden einer Stentstütze nach Anspruch 1, **dadurch gekennzeichnet, dass** der Schritt des Vernähens der Wicklungen auf den Stentabschnitten der Stentstütze ferner umfasst, dass die Wicklungen an den Rändern der Stentabschnitte vernäht werden können.

4. Verfahren zum Binden einer Stentstütze nach Anspruch 3, **dadurch gekennzeichnet, dass** der Schritt des Vernähens einer Wicklung auf den Stentabschnitten der Stentstütze ferner umfasst, dass die Wicklung in der mittleren Position der Ränder der Stentabschnitte vernäht werden kann.

5. Verfahren zum Binden einer Stentstütze nach Anspruch 3, **dadurch gekennzeichnet, dass** für die Stentstütze mit kleinem Durchmesser der doppelsträngige Bindedraht verwendet wird, um den Scheitelpunkt des Randes an dem ersten Abschnitt der Stentröhre zu binden.

6. Verfahren zum Binden einer Stentstütze nach Anspruch 3, **dadurch gekennzeichnet, dass** der Scheitelpunkt des Randes des Stentabschnitts an seinem distalen Ende mit einer Wicklung vernäht wird oder die Scheitelpunkte der benachbarten Ränder mit 2 bis 3 Wicklungen vernäht werden.

7. Verfahren zum Binden einer Stentstütze nach Anspruch 3, **dadurch gekennzeichnet, dass** der Schritt des Vernähens der Wicklungen auf den Stentabschnitten der Stentstütze ferner umfasst, dass die Wicklungen an jedem Rand oder an jedem zweiten Rand der Stentabschnitte vernäht werden.

8. Verfahren zum Binden einer Stentstütze nach einem der Ansprüche 1 bis 7, **dadurch gekennzeichnet, dass** die doppelsträngigen Bindedrähte gleich lang oder unterschiedlich lang sein können.

9. Verfahren zum Binden einer Stentstütze nach einem der Ansprüche 1 bis 7, **dadurch gekennzeichnet, dass** der doppelsträngige Bindedraht ein geschlossener Draht sein kann oder ein einziger Strang mit einer Wicklung an den beiden Enden sein kann oder doppelsträngig sein kann.

10. Verfahren zum Binden einer Stentstütze nach Anspruch 6, **dadurch gekennzeichnet, dass** die Ausstoßröhre in dem Abgabesystem, das verwendet wird, um die Stentstütze abzugeben und auszustoßen, ebenfalls mit einer Wicklung vernäht wird.

## Revendications

1. Procédé de liaison d'une endoprothèse (1), comprenant un fil de liaison (50) et un fil de commande (60),
**caractérisé par** les étapes suivantes :
suturer des spires (502) sur les sections de stent (10) de l'endoprothèse (1) ;
lier de manière circonférentielle un fil de liaison double brin (50) à travers les spires (502) sur l'endoprothèse (1) ; et
poser le fil de commande (60) à travers les deux extrémités du fil de liaison double brin (50).

2. Procédé de liaison d'une endoprothèse selon la revendication 1, **caractérisé en ce qu'**après l'enroulement d'un cercle autour de l'endoprothèse, le fil de liaison double brin est maintenu en position par le fil de commande qui s'étend à travers un cercle recouvrant formé par l'extrémité de début et l'extrémité de fin du fil de liaison de sorte que le diamètre de l'endoprothèse est limité à un cercle dont le périmètre est égal à la longueur du fil de liaison.

3. Procédé de liaison d'une endoprothèse selon la revendication 1, **caractérisé en ce que** l'étape consistant à suturer des spires sur les sections de stent de l'endoprothèse comprend en outre le fait que lesdites spires peuvent être suturées sur les bords des sections de stent.

4. Procédé de liaison d'une endoprothèse selon la revendication 3, **caractérisé en ce que** l'étape consistant à suture une spire sur les sections de stent de l'endoprothèse comprend en outre le fait que la spire peut être suturée dans la position centrale des bords des sections de stent.

5. Procédé de liaison d'une endoprothèse selon la revendication 3, **caractérisé en ce que** pour l'endoprothèse de petit diamètre, le fil de liaison double brin est utilisé pour lier le sommet du bord sur la première section du tube de stent.

6. Procédé de liaison d'une endoprothèse selon la revendication 3, **caractérisé en ce que** le sommet du bord sur la section de stent à son extrémité distale est suturé avec une spire, ou les sommets des bords adjacents sont suturés avec 2 à 3 spires.

7. Procédé de liaison d'une endoprothèse selon la revendication 3, **caractérisé en ce que** l'étape consistant à suturer des spires sur les sections de stent de l'endoprothèse comprend en outre le fait que les spires sont suturées sur chaque bord ou sur un bord sur deux des sections de stent.

8. Procédé de liaison d'une endoprothèse selon l'une quelconque des revendications 1 à 7, **caractérisé en ce que** les fils de liaison double brin peuvent être de longueur identique ou de longueur différente.

9. Procédé de liaison d'une endoprothèse selon l'une quelconque des revendications 1 à 7, **caractérisé en ce que** le fil de liaison double brin peut être un fil fermé ou peut être un seul brin avec une spire aux deux extrémités ou peut être à double brin.

10. Procédé de liaison d'une endoprothèse selon la revendication 6, **caractérisé en ce que** le tube d'éjection dans le système de déploiement utilisé pour déployer et éjecter l'endoprothèse est également suturé avec une spire.
